# EUROPEAN PATENT APPLICATION

(11) **EP 2 128 272 A1**
(43) Date of publication of application: **02.12.2009**
(21) Application number: 07829310.7
(22) Date of filing: 05.10.2007
(51) Int. Cl.: C12Q 1/68, C07K 17/00, C12M 1/00, C12N 15/09, G01N 33/53, G01N 37/00, A61K 45/00, A61P 35/00, A61P 43/00

(54) **GENE/PROTEIN MARKER FOR PREDICTION OR DIAGNOSIS OF PHARMACOLOGICAL EFFICACY OF AURORA A INHIBITOR**

(30) Priority: 05.10.2006 JP 2006273945
(71) Applicant: BANYU PHARMACEUTICAL CO., LTD., Tokyo 102-8667 (JP)
(72) Inventor: HASAKO, Shinichi, Tsukuba-shi Ibaraki 300-2611 (JP); ICHIKAWA, Koji, Tsukuba-shi Ibaraki 300-2611 (JP); KOTANI, Hidehito, Tsukuba-shi Ibaraki 300-2611 (JP); MIKI, Satomi, Tsukuba-shi Ibaraki 300-2611 (JP); MIYAMA, Katsuyoshi, Tsukuba-shi Ibaraki 300-2611 (JP); SHIMOMURA, Toshiyasu, Tsukuba-shi Ibaraki 300-2611 (JP); TAKAHASHI, Kazuhiko, Tsukuba-shi Ibaraki 300-2611 (JP); YAMANAKA, Kazunori, Tsukuba-shi Ibaraki 300-2611 (JP)
(74) Representative: Hussain, Deeba
(86) International application number: PCT/JP2007/069572
(87) International publication number: WO 2008/041767

(57) **Abstract**

[PROBLEMS] To provide: a gene marker or a protein marker for detecting whether or not an Aurora A inhibitor acts in a living body in an Aurora A-specific manner when the Aurora A inhibitor is administered to the living body; and a method for predicting or diagnosing the pharmacological efficacy of an Aurora A inhibitor by using the gene marker or the protein marker. [MEANS FOR SOLVING PROBLEMS] A gene/protein marker for use in the prediction or diagnosis of the pharmacological efficacy of an Aurora A inhibitor, wherein the gene is a gene selected from the group consisting of Aurora B, Histone H3, BIRC5, Pry1, DLG7, TACC3 and KNTC2 or a gene having substantially the same function as that of the gene; and a method for predicting or diagnosing the pharmacological efficacy of an Aurora A inhibitor by using the gene/protein marker.

## Description

### TECHNICAL FIELD

The present invention relates to a gene marker and a protein marker for predicting or determining the pharmacological efficacy of an Aurora A inhibitor. The invention also relates to a method for predicting or determining the pharmacological efficacy of an Aurora A inhibitor by using the gene marker or the protein marker.

### BACKGROUND ART

The recent development of human genome projects has rapidly promoted pharmacogenomics in which the individual difference in drug sensitivity is understood on a gene level and is applied to drug development. Success, if any, in analysis and determination of the individual difference in drug sensitivity on a gene level could enable tailor-made medicine in which specific drug administration is limited to only the patients who are expected to surely enjoy the pharmacological efficacy of the drug, suitably from the initial stage of therapy. As a result, it could save patients from being forced to take any useless burden, and could contribute toward reduction in medical costs that tend to increase these days. Above all, many anticancer agents often have extremely strong side effects, with which, therefore, tailor-made medicine is strongly desired.

Molecular level analysis of individual disease-related genes, if possible, enables drug administration suitable to the disease mechanism in case where one and the same disease is caused by different mechanisms. According to such drug administration mode, it may be possible to evade any side effects and to attain effective therapy and disease prevention.

On the other hand, Aurora A exists over a wide variety of species from yeast to human, and is known as a kinase to play an indispensable role for cell division control. Aurora A is a kinase of 403 amino acids, isolated by Kimura et al. (Non-Patent Reference 1), Sen et al. (Non-Patent Reference 2) and Zhou et al. (Non-Patent Reference 3); and this may be referred to also as Aik, STK15 or ARK1 (NM_003600, NM_198433, NM_198434, NM_198435, NM_198436 and NM_198437). Sen et al. have found that Aurora A is amplified highly frequently in breast cancer cells and have identified it as a gene positioned at chromosome 20q13; Zhou et al. have found that Aurora A is strongly expressed not only in breast cancer cells but also in ovarian cells, colon cells, prostatic cells, neuroblastomas, etc. Further, Zhou et al. have considered that Aurora A could function as a cancer gene, since in NIH3T3 cells with Aurora A over-expression therein, centrosomal abnormal amplification and transformation have occurred. Accordingly, studies of Aurora A as a target gene for anticancer agents are being promoted, and, for example, Patent Reference 1 discloses quinazoline derivatives having an Aurora (especially Aurora A) kinase inhibiting activity.

Patent Reference 1: JP-T 2005-525307,
Non-Patent Reference 1: J. Biol. Chem., Vol. 272, p. 13766, 1977,
Non-Patent Reference 2: Oncogene, Vol. 14, p. 2195, 1997,
Non-Patent Reference 3: Nature Genetics, Vol. 20, p. 189, 1998.

### DISCLOSURE OF THE INVENTION

### PROBLEMS THAT THE INVENTION IS TO SOLVE

At present, however, in case where an Aurora A inhibitor is administered to a living body, a marker capable of detecting the effect of the Aurora A inhibitor is as yet unknown.

The present invention has been made in consideration of the above-mentioned prior-art problems, and its one object is to provide a gene marker and a protein marker capable of detecting, in case where an Aurora A inhibitor is administered to a living body, as to whether or not the inhibitor could act on the living body in an Aurora A-specific manner. Another object of the invention is to provide a method for predicting or determining the pharmacological efficacy of an Aurora A inhibitor by using the gene marker or the protein marker.

### MEANS FOR SOLVING THE PROBLEMS

The present inventors have assiduously studied for the purpose of attaining the above-mentioned objects and, as a result, have found that Aurora A inhibitor administration changes the expression level of Aurora B, Histone H3, BIRC5, PRC1, DLG7, TACC3 and KNTC2 genes in a tissue, and have completed the invention.

Specifically, the gene marker of the invention is a gene marker for predicting or determining the pharmacological efficacy of an Aurora A inhibitor, and is **characterized in that** the gene is at least one gene selected from a group consisting of Aurora B, Histone H3, BIRC5, PRC1, DLG7, TACC3 and KNTC2. The gene marker makes it possible to predict or determine the pharmacological efficacy of an Aurora A inhibitor in a simplified manner and in a noninvasive manner.

The gene marker of the invention is a gene marker for determining the healing result of cancer, and is **characterized in that** the gene is at least one gene selected from a group consisting of Aurora B, Histone H3, BIRC5, PRC1, DLG7, TACC3 and KNTC2. The gene marker makes it possible to evaluate the healing result and progress of cancer with an Aurora A inhibitor in a simplified manner and in a noninvasive manner.

The cancer diagnostic kit of the invention is characterized by containing a PCR primer capable of detecting at least one gene selected from a group consisting of Aurora B, Histone H3, BIRC5, PRC1, DLG7, TACC3 and KNTC2. The cancer diagnostic kit enables cancer diagnosis and cancer curative progress evaluation with an Aurora A inhibitor in a simplified manner and in a noninvasive manner. As the PCR primer, usable is a primer set comprising PCR primers of SEQ ID NO. 1 and 2, a primer set comprising PCR primers of SEQ ID NO. 4 and 5, a primer set comprising PCR primers of SEQ ID NO. 7 and 8, a primer set comprising PCR primers of SEQ ID NO. 10 and 11, or a primer set comprising PCR primers of SEQ ID NO. 13 and 14.

The DNA microarray of the invention comprises at least one gene selected from a group consisting of Aurora B, Histone H3, BIRC5, PRC1, DLG7, TACC3 and KNTC2 or a partial nucleotide of the gene, and is **characterized in that** it is used for predicting or determining the effect of an Aurora A inhibitor. Using the DNA microarray makes it possible to evaluate plural genes including the gene marker of the invention all at the same time, thereby enabling rapid prediction and determination of pharmacological efficacy.

The protein marker of the invention is a protein marker for predicting or determining the pharmacological efficacy of an Aurora A inhibitor, and is **characterized in that** the protein is at least one gene selected from a group consisting of Aurora B, Histone H3, BIRC5, PRC1, DLG7, TACC3 and KNTC2. The protein marker makes it possible to determine the expression level on a protein level, thereby enabling prediction and determination of pharmacological efficacy not requiring gene isolation.

The protein marker of the invention is a protein marker for determining the healing result of cancer, and is **characterized in that** the protein is at least one protein selected from a group consisting of Aurora B, Histone H3, BIRC5, PRC1, DLG7, TACC3 and KNTC2. The protein marker makes it possible to determine the expression level on a protein level, thereby enabling evaluation of the healing result of cancer not requiring gene isolation.

The cancer diagnostic kit of the invention is characterized by containing an antibody capable of detecting the above-mentioned protein marker.

Further, the antibody array of the invention comprises an antibody to at least one protein selected from a group consisting of Aurora B, Histone H3, BIRC5, PRC1, DLG7, TACC3 and KNTC2, and is **characterized in that** it is used for predicting or determining the effect of an Aurora A inhibitor. Using the antibody array makes it possible to evaluate plural proteins including the protein marker of the invention all at the same time, thereby enabling rapid prediction and determination of pharmacological efficacy.

The method for predicting or determining the effect of an Aurora A inhibitor of the invention comprises a detection step of detecting at least one gene selected from a group consisting of Aurora B, Histone H3, BIRC5, PRC1, DLG7, TACC3 and KNTC2 in a tissue derived from a test subject to which an Aurora A inhibitor has been administered, and a comparison step of comparing the expression level before and after the Aurora A inhibitor administration. The method makes it possible to predict or determine the pharmacological efficacy of an Aurora A inhibitor in a simplified manner and in a noninvasive manner.

The method for predicting or determining the effect of an Aurora A inhibitor of the invention is **characterized in that** the detection means for the detection is a PCR or DNA microarray.

The method for predicting or determining the effect of an Aurora A inhibitor of the invention is characterized by comprising a detection step of detecting at least one protein selected from a group consisting of Aurora B, Histone H3, BIRC5, PRC1, DLG7, TACC3 and KNTC2 in a tissue derived from a test subject to which an Aurora A inhibitor has been administered, and a comparison step of comparing the expression level before and after the Aurora A inhibitor administration. The method makes it possible to predict or determine the pharmacological efficacy of an Aurora A inhibitor in a simplified manner and in a noninvasive manner.

The method for predicting or determining the effect of an Aurora A inhibitor of the invention is **characterized in that** the detection means for the detection is an anti-Aurora B antibody, an anti-Histone H3 antibody, an anti-BIRC5 antibody, an anti-PRC1 antibody, an anti-DLG7 antibody, an anti-TACC3 antibody or an anti-KNTC2 antibody.

The method for predicting or determining the effect of an Aurora A inhibitor of the invention is **characterized in that** the tissue is blood, skin, hair root, oral mucosa, digestive tract, bone marrow or cancer tissue. Using the tissue may facilitate sample preparation, therefore enabling noninvasive prediction or determination of pharmacological efficacy.

### EFFECT OF THE INVENTION

The invention has made it possible to provide a gene marker and a protein marker capable of detecting whether or not an Aurora A inhibitor acts in a living body in an Aurora A-specific manner when the Aurora A inhibitor is administered to the living body; and the marker expression as an index enables prediction or determination of the pharmacological efficacy of an Aurora A inhibitor in a simplified manner and in a noninvasive manner.

### BEST MODE FOR CARRYING OUT THE INVENTION

Preferred embodiments of the invention are described in detail hereinunder.

"Aurora A gene" in the invention includes genes with substitution, deletion, addition or insertion of one or more bases, having the same-level biological function as that of an Aurora A gene and coding for a kinase activity-having protein. The gene is not specifically defined in point of its sequence so far as it codes for the protein; however, the homology is preferably at least 50 %, more preferably at least 70 %, even more preferably at least 80 %, still more preferably at least 90 % (for example, 91, 92, 93, 94, 95, 96, 97, 98 or 99 % or more).

The Aurora A gene in the invention also includes a nucleic acid capable of hybridizing with an Aurora A gene under a stringent condition. "Hybridizing under a stringent condition" as referred to herein means that the two nucleic acid fragments hybridize with each other under the hybridization condition described in Molecular Cloning: A Laboratory Manual, 2nd Edition, Cold Spring Harbor (1989), 9.47-9.62 and 11.45-11.61. More concretely, for example, the process under the condition comprises 6.0 × SSC hybridization at about 45°C followed by 2.0 × SSC washing at 50°C. For stringency selection, the salt concentration in the washing step may fall within a range of from about 2.0 × SSC at 50°C of low stringency to about 0.2 × SSC at 50°C of high stringency. Further, the temperature in the washing step may be elevated from room temperature of about 22°C under a low stringency condition up to about 65°C under a high stringency condition.

Aurora A may be referred to as Aik, Aurora-2, AIRK1, STK15, BTAK, ARK1, IAK1 or Ayk1; and any of these has the same meaning of Aurora A in the invention.

"Aurora A inhibitor" in the invention is a substance or a molecule that inhibits the activity of an Aurora A kinase. The inhibitor is not specifically defined in point of the molecular species thereof, so far as it is a molecule that functions as an inhibitor to Aurora A. Concretely, for example, it includes low-molecular compounds, proteins and peptides. The low-molecular compounds are not also specifically defined in point of the type thereof; and concretely, for example, they include natural compounds, organic compounds or inorganic compounds. Specific examples of the low-molecular compounds include, for example, MLN8054, ZM447439, 6-((4-(2,3-difluorobenzoyl)piperazin-1-yl)methyl)-N-thiazol-2-ylpyridin-2-amine, 6-((4-(2-fluoro-3-(trifluoromethyl)benzoyl)piperazin-1-yl)methyl)-N-1H-pyrazol-3-ylpyrazin-2-amine, and 6-((4-(3-chloro-2-fluorobenzoyl)piperazin-1-yl) methyl)-N-thiazol-2-ylpyridin-2-amine. The peptides are not also specifically defined in point of the type thereof, and may have any desired number of amino acids.

The disorders to which the inhibitor is directed are not specifically defined, so far as they are disorders to be caused by malfunction of Aurora A or molecules on the intercellular information transmission pathway via Aurora A. The disorders to which the inhibitor is directed include, for example, brain tumor, pharyngeal cancer, laryngeal cancer, thymoma, mesothelioma, breast cancer, lung cancer, stomach cancer, esophageal cancer, colon cancer, hepatocellular cancer, pancreatic cancer, pancreatic endocrine tumor, bile duct cancer, gallbladder cancer, penile cancer, renal pelvic/ureteral cancer, renal cell cancer, testicular tumor, prostate cancer, bladder cancer, vulvar cancer, uterine cancer, uterine sarcoma, trophoblastic disease, vaginal cancer, breast cancer, ovarian cancer, ovarian germ cell tumor, malignant melanoma, mycosis fungoides, skin cancer, soft tissue sarcoma, malignant lymphoma, myelodysplastic syndrome, multiple myeloma or leukemia.

"At least one gene selected from a group consisting of Aurora B (NM_004217), Histone H3 (NM_002107 or NM_005324), BIRC5 (NM_001012270, NM_001012271 or NM_001168), PRC1 (NM_003981, NM_199413 or NM_199414), DLG7 (NM_014750), TACC3 (NM_006342) and KNTC2 (NM_006101)" in the invention includes genes with substitution, deletion, addition or insertion of one or more bases, coding for a protein having the same-level biological function as that of at least one gene selected from a group consisting of Aurora B, Histone H3, BIRC5, PRC1, DLG7, TACC3 or KNTC2. The gene is not specifically defined in point of its sequence so far as it codes for the protein; however, the homology is preferably at least 50 %, more preferably at least 70 %, even more preferably at least 80 %, still more preferably at least 90 % (for example, 91, 92, 93, 94, 95, 96, 97, 98 or 99 % or more).

At least one gene selected from a group consisting of Aurora B, Histone H3, BIRC5, PRC1, DLG7, TACC3 or KNTC2 in the invention includes a nucleic acid capable of hybridizing with any one gene selected from a group consisting of Aurora B, Histone H3, BIRC5, PRC1, DLG7, TACC3 or KNTC2 under a stringent condition and coding for a protein having the same-level activity as that of the gene. "Hybridizing under a stringent condition" as referred to herein means that the two nucleic acid fragments hybridize with each other under the hybridization condition described in Molecular Cloning: A Laboratory Manual, 2nd Edition, Cold Spring Harbor (1989), 9.47-9.62 and 11.45-11.61. More concretely, for example, the process under the condition comprises 6.0 × SSC hybridization at about 45°C followed by 2.0 × SSC washing at 50°C. For stringency selection, the salt concentration in the washing step may fall within a range of from about 2.0 × SSC at 50°C of low stringency to about 0.2 × SSC at 50°C of high stringency. Further, the temperature in the washing step may be elevated from room temperature of about 22°C under a low stringency condition up to about 65°C under a high stringency condition. Specific examples of the nucleic acid capable of hybridizing with any one gene selected from a group consisting of Aurora B, Histone H3, BIRC5, PRC1, DLG7, TACC3 or KNTC2 under a stringent condition include, for example, nucleic acids having polymorphism such as SNP or RFLP in the gene.

### (1) Gene Marker:

The gene marker of the invention is a gene marker for predicting or determining the pharmacological efficacy of an Aurora A inhibitor, and is **characterized in that** the gene is at least one gene selected from a group consisting of Aurora B, Histone H3, BIRC5, PRC1, DLG7, TACC3 and KNTC2, or a gene having substantially the same-level function as that of the gene.

"Gene marker" of the invention is meant to indicate an index of the pharmacological efficacy or the effect to a living body of an Aurora A inhibitor that is to be evaluated; and concretely, it includes genes or their related substances (e.g., DNA and RNA and their fragments), of which the expression level or the activity changes depending on the action of an Aurora A inhibitor.

The gene marker of the invention also includes a polynucleotide comprising a part of Aurora B, Histone H3, BIRC5, PRC1, DLG7, TACC3 and KNTC2. The polynucleotide may also be RNA transcribed from an Aurora B, Histone H3, BIRC5, PRC1, DLG7, TACC3 or KNTC2 gene, cDNA produced from them, as well as a synthetic nucleic acid having a sequence derived from these genes.

The inventors have confirmed that the expression of the gene marker of the invention increases owing to Aurora A inhibitor administration. The inventors have also confirmed that, in case where the expression of Aurora A and Aurora B is inhibited by the use of siRNA, then the expression of the gene to be the gene marker increases only when the expression of Aurora A is inhibited. Specifically, the inventors have confirmed that the gene marker of the invention functions as a selective and specific marker to an Aurora A inhibitor. The cytotypic expression of Aurora A inhibition is induction of cell division delay and mitotic catastrophe caused by activation of cell division checkpoints. On the other hand, the cytotypic expression of Aurora B inhibition is multiploidization of cell nuclei by cell division checkpoint cancellation and cytoplasmic division inhibition. Accordingly, the Aurora B inhibition acts in the direction that cancels the effect of Aurora A inhibition. Aurora A and Aurora B have high homology to each other; and many ATP-competitive Aurora inhibitors inhibit both Aurora A and Aurora B. Specifically, it may be said that an Aurora A-selective marker is an important tool for screening and evaluation of candidate compounds in developing Aurora A-specific inhibitors; and in clinical technology, the Aurora A-selective marker is useful as a marker for prediction or determination of pharmacological efficacy.

The gene marker of the invention increases its expression in the tissue of a living body by administration of an Aurora A inhibitor to the living body. In case of prediction or determination of the pharmacological efficacy of an Aurora A inhibitor, the expression level of the gene marker in a tissue in which the gene marker has been expressed may be measured; and from the viewpoint of easy sample availability, the expression level in a blood or skin tissue sample is preferably measured.

The method of measuring the expression level of the gene marker of the invention is not specifically defined. Concretely, for example, the expression level may be quantified by measuring the mRNA amount according to a northern blotting method or a quantitative RT-PCR method or with a DNA microarray.

The probe for use in the northern blotting method may be any probe capable of detecting the gene marker of the invention, Aurora B, Histone H3, BIRC5, PRC1, DLG7, TACC3 or KNTC2 gene. Concretely, for the probe, usable is a partial sequence or total sequence of the base sequence of those genes. The number of the bases of the probe is not also specifically defined; however, preferred is a nucleic acid having a length of at least continuous 20 bases, more preferably at least 40 bases, even more preferably at least 60 bases, still more preferably at least 80 bases. If desired, the probe may be labeled for detection. Concretely, it may be labeled with a radioisotope of ³²P, ¹⁴C, ¹²⁵I, ³H, ³⁵S or the like, or may be labeled with biotin, fluorescent dye, enzyme, gold colloid or the like.

The primer to be used in the above quantitative RT-PCR method may be any primer capable of detecting the gene marker of the invention, Aurora B, Histone H3, BIRC5, PRC1, DLG7, TACC3 or KNTC2 gene; and the number of the bases constituting it is not specifically defined but may be suitably determined depending on the base sequence of the primer, the base sequence of the gene to be isolated, etc. In general, it comprises preferably from 10 to 60 continuous bases, more preferably from 15 to 30 bases. The base sequence is determined based on the base sequence of the Aurora B, Histone H3, BIRC5, PRC1, DLG7, TACC3 or KNTC2 gene to be detected.

In case where the expression level of the gene marker of the invention is measured according to the above DNA microarray method, a DNA microarray in which at least one of Aurora B, Histone H3, BIRC5, PRC1, DLG7, TACC3 and KNTC2 genes or a partial nucleic acid of the gene is spotted, is prepared, and used for the measurement.

The gene marker of the invention is used for predicting and determining the pharmacological efficacy of an Aurora A inhibitor. Specifically, an Aurora A inhibitor is administered to a living body, then the expression level of the gene marker therein is measured; and in case where the expression level has increased as compared with the expression level before the administration, it may be recognized that the inhibitor acts specifically to Aurora A, therefore exhibiting a predetermined pharmacological efficacy. Specifically, in case where the expression level has increased after Aurora A inhibitor administration, it may be predicted that the inhibitor can exhibit its pharmacological efficacy even though the pharmacological efficacy of the inhibitor could not be on a visible level (for example, for relieving cancer symptom) or even in a case where the cancer tissue is extremely small and its diagnosis is difficult according to a conventional diagnostic method such as X-ray photography. When the clinical test in a stage of developing an Aurora A inhibitor as a pharmaceutical agent is directed to healthy persons, the pharmacological efficacy of the pharmaceutical agent can be evaluated based on the expression level of the gene marker of the invention as an index thereof.

The gene marker of the invention can also be used in determining the healing result of cancer. Specifically, in case where an Aurora A inhibitor is administered to a living body as an anticancer agent for cancer treatment, it must be confirmed that the cancer tissue has actually reduced or the number of the cancer cells has actually reduced in order to confirm as to whether or not the anticancer agent could be effective. The confirmation requires examination with exposure to radiations such as X-ray tomography or X-ray imaging photography, or requires examination with load to patients such as endoscopy or biopsy. However, the gene marker of the invention requires only a simple examination of such that only an extremely small amount of a part of a body tissue such as blood or skin is collected, and the expression level of the gene marker in the tissue is measured. Accordingly, in place of the examination with X-ray tomography, X-ray imaging photography, endoscopy or biopsy, or as a preliminary examination for the examination, the pharmacological efficacy of an Aurora A inhibitor can be determined with neither pain nor load given to patients.

### (2) Protein Marker:

The protein marker of the invention is a protein marker for predicting or determining the pharmacological efficacy of an Aurora A inhibitor, and is **characterized in that** the protein is at least one selected from a group consisting of Aurora B, Histone H3, BIRC5, PRC1, DLG7, TACC3 and KNTC2, or one having substantially the same-level function as that of the protein.

The "protein marker" of the invention is one that can be an index to the pharmacological efficacy of an Aurora A inhibitor to be analyzed and the effect thereof to living bodies; and concretely, it includes a protein or its related substances (e.g., partial peptides) of which the expression level and the activity vary depending on the effect of the Aurora A inhibitor thereto.

The inventors have confirmed that the expression level of not only the gene coding for those proteins but also the protein itself increases by administration of an Aurora A inhibitor. The inventors have also confirmed that, in case where the expression of Aurora A and Aurora B is inhibited by the use of siRNA, then the expression of the protein to be the protein marker increases only when the expression of Aurora A is inhibited. Specifically, the protein marker of the invention functions as a selective and specific marker to an Aurora A inhibitor.

The expression level of the protein marker of the invention increases in the tissue of a living body by administration of an Aurora A inhibitor. In case where the pharmacological efficacy of an Aurora A inhibitor is predicted or determined, the expression level of the protein marker in a desired tissue in which the protein marker has been expressed may be measured; and from the viewpoint of easy sample availability, the expression level in blood or skin tissue is preferably measured.

The protein marker of the invention includes a protein having substantially the same-level function as that of at least one protein selected from a group consisting of Aurora B, Histone H3, BIRC5, PRC1, DLG7, TACC3 and KNTC2. The protein includes those derived from a protein of Aurora B, Histone H3, BIRC5, PRC1, DLG7, TACC3 or KNTC2 through substitution, addition, deletion or insertion at one or more amino acids constituting the amino acid sequence of the protein, and having the same-level activity as that of those proteins. The activity on the same level means as follows: The protein having substantially the same-level function as that of Aurora A is a protein having the same kinase activity as that of Aurora B; the protein having substantially the same-level function as that of Histone H3 is a protein having the same-level activity as that of Histone H3; the protein having substantially the same-level function as that of BIRC5 is a protein having the same-level activity as that of BIRC5; the protein having substantially the same-level function as that of PRC1 is a protein having the same-level activity as that of PRC1; the protein having substantially the same-level function as that of DLG7 is a protein having the same-level activity as that of DLG7; the protein having substantially the same-level function as that of TACC3 is a protein having the same-level activity as that of TACC3; and the protein having substantially the same-level function as that of KNTC2 is a protein having the same-level activity as that of KNTC2. The protein with substitution, deletion, addition or insertion of one or more amino acids in the amino acid sequence constituting the Aurora B, Histone H3, BIRC5, PRC1, DLG7, TACC3 or KNTC2 protein is not specifically defined in point of the sequence thereof; however, the homology is preferably at least 50 %, more preferably at least 70 %, even more preferably at least 80 %, still more preferably at least 90 % (for example, 91, 92, 93, 94, 95, 96, 97, 98 or 99 % or more).

The method for measuring the expression level of the protein marker of the invention is not specifically defined. Concretely, for example, the expression level may be determined by measuring the amount by mass of the protein produced, according to a western blotting method or an ELISA method or by the use of a protein chip.

The western blotting method may be attained according to a method known to those skilled in the art. Concretely, for example, the total protein of the tissue collected from a living body to which an Aurora A inhibitor has been administered is developed in SDS-PAGE, and transferred onto a nitrocellulose membrane, a PVDF membrane or the like. Next, an antibody to the protein marker of the invention is added to and reacted with the membrane, and further a secondary antibody is added to and reacted with it, thereby detecting the secondary antibody to detect the protein marker. The total protein of the tissue collected from the living body may be immunized and precipitated with an antibody to the protein marker, then the protein obtained as a precipitate may be developed in SDS-PAGE, and may be detected according to a western blotting method.

The antibody to be used in the western blotting method may be any antibody capable of detecting the protein marker of the invention, a protein of Aurora B, Histone H3, BIRC5, PRC1, DLG7, TACC3 or KNTC2, and it may be a polyclonal antibody or a monoclonal antibody. The polyclonal antibody may be prepared according to a method known to those skilled in the art, and for example, it may be prepared according to the method mentioned below. Specifically, for example, a mammal of mouse, rat, hamster, guinea pig, rabbit or the like is immunized with an antigen optionally along with a Freund's adjuvant, and the intended polyclonal antibody may be collected from the serum of the thus-immunized animal.

The monoclonal antibody may also be prepared as follows: For example, a mammal of mouse, rat, hamster, guinea pig, rabbit or the like is immunized with an antigen optionally along with a Freund's adjuvant. Next, an antibody-producing cell collected from the immunized animal is hybridized with a myeloma cell not having autoantibody producibility, thereby preparing a hybridoma (fused cell); then the hybridoma is cloned, and a clone that produces a monoclonal antibody having a specific affinity to the antigen used for the mammal immunization is selected, thereby preparing the intended monoclonal antibody. Further concretely, an antigen is injected or transplanted subcutaneously, intramuscularly, intravenously or intraabdominally once or a few times into a mammal optionally along with a Freund's adjuvant for mammal immunization. In general, the immunization is effected once to four times or so at intervals of from 1 to 14 days from the first immunization, and after 1 to 5 days or so from the final immunization, an antibody-producing cell is collected from the immunized mammal.

The hybridoma (fused cell) of secreting a monoclonal antibody may be prepared according to a conventional method. Specifically, the antibody-producing cell in the spleen, the lymph node, the bone marrow, the tonsil or the like, preferably in the spleen collected from the mammal thus immunized according to the above-mentioned method is fused with a myeloma cell derived from mouse, rat, human or the like and not having autoantibody producibility, thereby preparing a hybridoma. The hybridoma clone capable of producing a monoclonal antibody may be screened as follows: The hybridoma is cultured on a micro-titer plate or the like, and the reactivity to immunogen of the culture supernatant in the well in which the hybridoma cell has grown is measured, for example, according to enzyme immunoassay such as RIA or ELISA.

A monoclonal antibody may be produced from the hybridoma, as follows: The hybridoma is cultured in-vitro, or in-vivo in the ascites or the like of mouse, rat, guinea pig, hamster, rabbit or the like, and then the intended monoclonal antibody may be isolated from the resulting culture supernatant or from the ascites of the mammal. The monoclonal antibody may be isolated and purified by processing the above-mentioned culture supernatant or ascites through saturated ammonium sulfate treatment, euglobulin precipitation, caproic acid treatment, caprylic acid treatment, ion-exchange chromatography (DEAE, DE52, etc.), or affinity column chromatography with an antiimmunoglobulin column, a protein A column or the like.

Using the polyclonal antibody or the monoclonal antibody produced according to the above-mentioned method, an antibody array may be prepared. Specifically, the antibody may be fixed on a membrane such as a nitrocellulose membrane or a PVDF membrane, or on a nitrocellulose-coated slide or glass substrate, using a microarray spotter.

On the other hand, at least one protein of Aurora B, Histone H3, BIRC5, PRC1, DLG7, TACC3 or KNTC2, the protein marker of the invention, may be fixed on a membrane such as a nitrocellulose membrane or a PVDF membrane, or on a nitrocellulose-coated substrate or glass substrate, using a microarray spotter, thereby preparing a protein array. In this case, the protein may be fixed directly, or may be fixed via a linker held between the protein and the membrane or the substrate. Fixation via a linker makes it possible to prepare a protein array having the activity of the protein not detracting from the stereostructure thereof.

The protein marker of the invention may be used for predicting or determining the pharmacological efficacy of an Aurora A inhibitor. Specifically, after an Aurora A inhibitor has been administered to a living body, the expression level of the protein marker is measured, and in case where the expression level has increased as compared with the expression level before administration, then it may be recognized that the inhibitor acts specifically to Aurora and exhibits the predetermined pharmacological efficacy. Concretely, when the expression level of the protein marker of the invention has increased after the administration of the Aurora A inhibitor, then it may be anticipated that the Aurora A inhibitor can exhibit its pharmacological efficacy even though the pharmacological efficacy of the inhibitor could not be on a visible level (for example, for relieving cancer symptom) or even in a case where the cancer tissue is extremely small and its diagnosis is difficult according to a conventional diagnostic method such as X-ray photography. When the clinical test in a stage of developing an Aurora A inhibitor as a pharmaceutical agent is directed to healthy persons, the effect of the pharmaceutical agent can be evaluated based on the expression level of the protein marker of the invention as an index.

The protein marker of the invention can also be used in determining the healing result of cancer. Specifically, in case where an Aurora A inhibitor is administered to a living body as an anticancer agent, it must be confirmed that the cancer tissue has actually reduced or the number of the cancer cells has actually reduced in order to confirm as to whether or not the anticancer agent could be effective. The confirmation requires examination with exposure to radiations such as X-ray tomography or X-ray imaging photography, or requires examination with load to patients such as endoscopy or biopsy. However, the protein marker of the invention requires only a simple examination of such that only an extremely small amount of a part of a body tissue such as blood or skin is collected, and the expression level of the protein marker in the tissue is measured. Accordingly, in place of the examination with X-ray tomography, X-ray imaging photography, endoscopy or biopsy, or as a preliminary examination for the examination, the pharmacological efficacy of an Aurora A inhibitor can be determined with neither pain nor load given to patients.

### (3) Cancer Diagnostic Kit:

The cancer diagnostic kit of the invention is characterized by containing an antibody capable of detecting the protein marker of the invention. The antibody is a polyclonal antibody or a monoclonal antibody to at least one protein selected from a group consisting of Aurora B, Histone H3, BIRC5, PRC1, DLG7, TACC3 or KNTC2 mentioned in the above, or to a protein having substantially the same-level function as that of the protein.

The cancer diagnostic kit of the invention may be provided with, for example, a reagent for antibody detection, a secondary antibody for antibody detection and a plate for reaction, in addition to the antibody capable of detecting the protein marker of the invention.

Using the cancer diagnostic kit of the invention makes it possible to rapidly and simply detect a protein marker in a tissue (for example, blood, skin) collected from a living body to which an Aurora A inhibitor has been administered; and by comparing the protein amount by mass before and after the administration, the pharmacological efficacy of the Aurora A inhibitor can be thereby predicted or determined.

### (4) Method for Predicting or Determining the Effect of Aurora A Inhibitor:

First described in a first method for predicting or determining the effect of an Aurora A inhibitor of the invention.

The first method for predicting or determining the effect of an Aurora A inhibitor of the invention is characterized by comprising a detection step of detecting at least one gene selected from a group consisting of Aurora B, Histone H3, BIRC5, PRC1, DLG7, TACC3 and KNTC2 or a gene having substantially the same-level function as that of the gene in the tissue derived from a test subject to which an Aurora A inhibitor has been administered, and a step of comparing the expression level before and after the Aurora A inhibitor administration.

The detection step in the invention is a step of detecting at least one gene selected from a group consisting of Aurora B, Histone H3, BIRC5, PRC1, DLG7, TACC3 and KNTC2 or a gene having substantially the same-level function as that of the gene (that is, the gene marker of the invention) in the tissue derived from a test subject to which an Aurora A inhibitor has been administered.

"Tissue" in the invention means the tissue derived from a subject to which an Aurora A inhibitor has been administered, and may be any one in which the gene marker to be detected is expressed; and its type is not specifically defined. However, from the viewpoint of easy sample availability, the tissue is preferably blood or skin tissue.

In this step, the tissue is collected from a living body to which an Aurora A inhibitor has been administered, and a DNA for gene marker detection is extracted from it according to a conventional method. The DNA to be extracted may be a genome DNA, or may also be a cDNA obtained from the extracted RNA through reverse transcription. Next, the obtained DNA is used for detection of a gene Aurora B, Histone H3, BIRC5, PRC1, DLG7, TACC3 or KNTC2. Not specifically defined, the detection method may be any quantitative one, concretely, for example, includes PCR or microarray.

After a gene Aurora B, Histone H3, BIRC5, PRC1, DLG7, TACC3 or KNTC2 has been detected in the detection step, the step is then followed by the comparison step of comparing the expression level before and after the Aurora A inhibitor administration. In the comparison step, the expression level of the gene Aurora B, Histone H3, BIRC5, PRC1, DLG7, TACC3 or KNTC2 detected in the detection step is compared with the expression level of that gene before administration of the Aurora A inhibitor. As a result of the comparison, in case where the expression level of the gene has increased as a result of the Aurora A inhibitor administration, then it may be determined that the Aurora A inhibitor administered to the administration subject, human body, acts on the human body according to a suitable mechanism process, and exhibits its administration effect thereto. The method is extremely advantageous as a method for determining the pharmacological efficacy of an Aurora A inhibitor in case where a cancer tissue is extremely small or where the pharmacological efficacy of the inhibitor could hardly be determined on the basis of the physical size of a cancer tissue as in the stage of a clinical test in drug development.

Next described is a second method for predicting or determining the effect of an Aurora A inhibitor of the invention.

The second method for predicting or determining the effect of an Aurora A inhibitor of the invention is characterized by comprising a detection step of detecting at least one protein selected from a group consisting of Aurora B, Histone H3, BIRC5, PRC1, DLG7, TACC3 or KNTC2 or a protein having substantially the same-level function as that of the protein in the tissue derived from a test subject to which an Aurora A inhibitor has been administered, and a comparison step of comparing the expression level before and after the Aurora A inhibitor administration.

The detection step in the invention is a step of detecting at least one protein selected from a group consisting of Aurora B, Histone H3, BIRC5, PRC1, DLG7, TACC3 or KNTC2 or a protein having substantially the same-level function as that of the protein (that is, the protein marker of the invention) in the tissue derived from a test subject to which an Aurora A inhibitor has been administered.

"Tissue" in the invention means the tissue derived from a subject to which an Aurora A inhibitor has been administered, and may be any one in which the protein marker to be detected is expressed; and its type is not specifically defined. However, from the viewpoint of easy sample availability, the tissue is preferably blood or skin tissue.

In this step, the tissue is collected from a living body to which an Aurora A inhibitor has been administered, and a protein for protein marker detection is extracted from it according to a conventional method, or a total protein including the protein is collected. Concretely, for example, a total protein including the protein is prepared as a solubilized sample so as to be applicable to detection in a western blotting method, and this may be used as the extract protein in this step. The total protein as a sample may be processed for immunoprecipitation using an antibody to the protein to be detected, whereby the protein alone may be extracted.

Next, the obtained protein is processed for detection of the intended protein Aurora B, Histone H3, BIRC5, PRC1, DLG7, TACC3 or KNTC2. Not specifically defined, the detection method may be any quantitative one. Concretely, for example, it includes a western blotting method and an ELISA method. Detection according to a western blotting method and an ELISA method requires an anti-Aurora B antibody, an anti-Histone H3 antibody, an anti-BIRC5 antibody, an anti-PRC1 antibody, an anti-DLG7 antibody, an anti-TACC3 antibody or an anti-KNTC2 antibody. The antibody may be a polyclonal antibody or a monoclonal antibody. The antibody may be prepared according to a conventional method, and for example, it may be prepared according to the method described hereinabove in the section of (2) protein marker.

After a protein Aurora B, Histone H3, BIRC5, PRC1, DLG7, TACC3 or KNTC2 has been detected in the detection step, the step is then followed by the comparison step of comparing the expression level before and after the Aurora A inhibitor administration. In the comparison step, the expression level of the protein Aurora B, Histone H3, BIRC5, PRC1, DLG7, TACC3 or KNTC2 detected in the detection step is compared with the expression level of that protein before administration of the Aurora A inhibitor. As a result of the comparison, in case where the expression level of the protein has increased as a result of the Aurora A inhibitor administration, then it may be determined that the Aurora A inhibitor administered to the administration subject, human body, acts on the human body according to a suitable mechanism process, and exhibits its administration effect thereto. The method is extremely advantageous as a method for determining the pharmacological efficacy of an Aurora A inhibitor in case where a cancer tissue is extremely small or where the pharmacological potency of the inhibitor could hardly be determined on the basis of the physical size of a cancer tissue as in the stage of a clinical test in drug development.

### EXAMPLES

The invention is described more concretely with reference to the following Examples, to which, however, the invention should not be limited.

### Test Examples 1 to 14

### (Gene Expression Analysis)

For identifying a PD marker capable of evaluating the effect of an Aurora A inhibitor, a group of genes of which the expression level can be specifically changed by an Aurora A inhibitor or Aurora A siRNA were searched for through microarray analysis. In the microarray analysis, used was the following samples with on a GeneChip array (U-133A: Affymetrix).

### 1. GeneChip Analysis in Treatment with Aurora A or Aurora B siRNA:

siRNA of Aurora A, Aurora B or luciferase (control) was introduced into HeLa-S3 cells through lipofection (concentration: 12.5 nM); and after 24 hours and 48 hours, the cells were collected to prepare RNA for microarray analysis. Concretely, the following 6 samples were collected.

Aurora A siRNA 24 hrs (Test Example 1)
Aurora A siRNA 48 hrs (Test Example 2)
Aurora B siRNA 24 hrs (Test Example 3)
Aurora B siRNA 48 hrs (Test Example 4)
Luciferase siRNA 24 hrs (Test Example 5)
Luciferase siRNA 48 hrs (Test Example 6)

### 2. GeneChip Analysis in Treatment with Aurora A Inhibitor:

An Aurora A inhibitor, 6-((4-(2,3-difluorobenzoyl)piperazin-1-yl)methyl)-N-thiazol-2-ylpyridin-2-amine (1, 3, 10 µM) was acted on HeLa-S3 cells; and after 24 hours and 48 hours, the cells were collected to prepare RNA for microarray analysis. As a control, used was DMSO. Concretely, the following 8 samples were collected.

Aurora A Inhibitor (1 µM) 24 hrs (Test Example 7)
Aurora A Inhibitor (1 µM) 48 hrs (Test Example 8)
Aurora A Inhibitor (3 µM) 24 hrs (Test Example 9)
Aurora A Inhibitor (3 µM) 48 hrs (Test Example 10)
Aurora A Inhibitor (10 µM) 24 hrs (Test Example 11)
Aurora A Inhibitor (10 µM) 48 hrs (Test Example 12)
DMSO 24 hrs (Test Example 13)
DMSO 48 hrs (Test Example 14)
Next, for extracting candidate genes of an Aurora A-specific PD marker, a group of genes satisfying the following three conditions were extracted. Concretely, a group of genes satisfying the following conditions (1) to (3) were extracted.
(1) Of the above samples on which 6-((4-(2,3-difluorobenzoyl)piperazin-1-yl)methyl)-N-thiazol-2-ylpyridin-2-amine had been acted as Test Examples 7 to 12, those of which the expression level significantly changed by at least 1.5 times based on the control DMSO in the above 2.
(2) Of the above samples treated with Aurora A siRNA for 24 hours or 48 hours (Test Examples 1 and 2), those of which the expression level significantly changed by at least 1.5 times based on the control luciferase in the above 1.
(3) Of the above samples treated with Aurora B siRNA (Test Examples 3 and 4), those of which the expression level did not change (but specifically changed with Aurora A) based on the control luciferase in the above 1.
(4) Genes reported to satisfy the above 1 to 3 and to have the function of Aurora A substrate or its related function.

As a result, as genes of which the expression level changed owing to Aurora A inhibition but did not change by Aurora B inhibition, BIRC5, KNTC2, PRC1, TACC3 and DLG7 were found out.

### Example 1

### (Expression Change of PD Marker Candidate Genes in case of Aurora A or Aurora B expression inhibition)

The samples of Test Examples 1 to 6 were confirmed for the expression change of PD marker candidate genes in real-time PCR. First, from the total DNA isolated from the cell, cDNA was prepared using a reverse transcriptase. The obtained cDNA was processed in real-time PCR, according to an ordinary protocol attached to a TaqMan reagent.

The primer sets used in real-time PCR are as follows. For every primer and probe, a human gene was used as the template.

**[Table 1]**

| Gene | Forward primer | Reverse primer | Probe |
|---|---|---|---|
| BIRC5 | CTTTCTTTGGAGGCAG CAGC (SEQ ID NO 1) | GGGCGAATCAAATCC ATCAT (SEQ ID NO 2) | CGCAGGGCTGAAGTC TGGCGTAA (SEQ ID NO 3) |
| PRC1 | TCTCATCTCCCCCATT CCTG (SEQ ID NO 4) | ACTCCCAATGTGGCT GGC (SEQ ID NO 5) | TGCCTGAGTTCTTCTA CCCCCGCA (SEQ ID NO 6) |
| KNTC2 | (SEQ ID NO [?]) AGAATTCCAAAGGTT ATGACTTTGAAA (SEQ ID NO 7) | GCCCTGTATTTGACAA GGCAG (SEQ ID NO 8) | AAGTTTAATCCCGAG GCTGGTGCCA (SEQ ID NO 9) |
| TACC3 | AAGAAGTGCGTGGAG GATTACCT (SEQ ID NO 10) | GCCTTCAGGGCTTGG TACCT (SEQ ID NO 11) | CAAGGATCACCCAGG AGGGCCA (SEQ ID NO 12) |
| DLG7 | GCTGGAGAGGAGACA TCAAGAAC (SEQ ID NO 13) | CCTGGTTGTAGAGGT GAAAAAGTAATC (SEQ ID NO 14) | TGCCAGACACATTTCT TTTGGTGGTAACC (SEQ ID NO 15) |

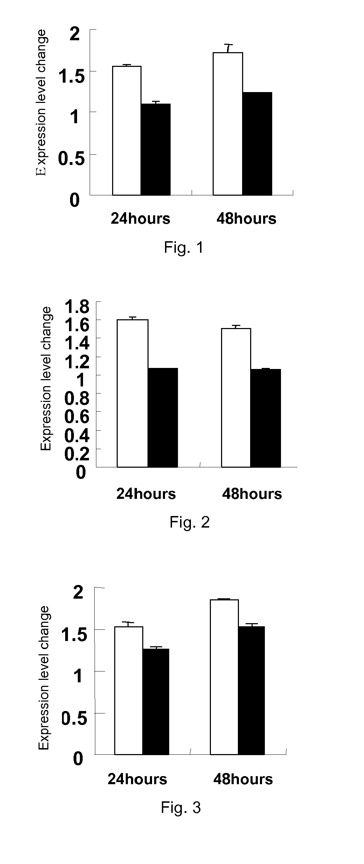

As in Figs 1 to 5, it has been confirmed that, in every case where Aurora A siRNA was acted on the samples, the candidate gene expression level increased for the reaction time of 24 hours and 48 hours. On the other hand, in the case where Aurora B siRNA was acted, there was found no change as in the case with Aurora A.

### Example 2

### (Expression Change of PD Marker Candidate Genes in case of treatment with Aurora A inhibitor)

A predetermined concentration of 6-((4-(2,3-difluorobenzoyl)piperazin-1-yl)methyl)-N-thiazol-2-ylpyridin-2-amine was acted on HeLa-S3 cells; and after 24 hours, the cells were collected, the total RNA was isolated and then converted into cDNA, and this was processed for real-time PCR in the same manner as in Example 1.

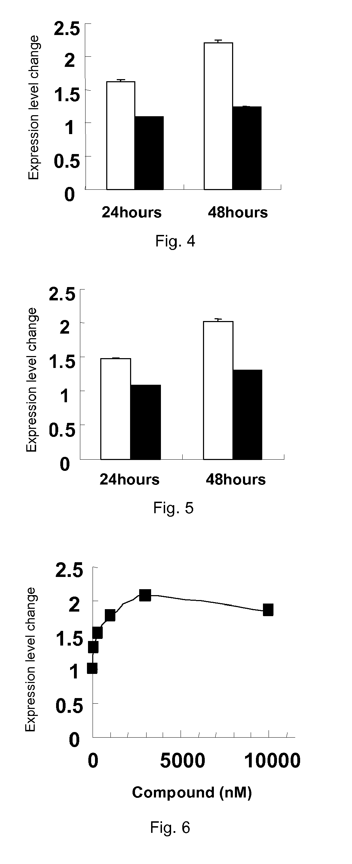

As in Figs. 6 to 10, it has been confirmed that the expression level of the PD marker candidate genes increased dependently on the Aurora A inhibitor concentration.

### Example 3

### (Expression Change of PD Marker Candidate Genes in planted cancer in case of continuous administration of Aurora A inhibitor to rat)

First, a cervical cancer strain HeLa-luc cells were subcutaneously transplanted to the back of an F344/N Jcl-rnu female nude rat. After 1 week, a gallbladder cancer nude rat was subjected to femoral vein cannulation operation; and using a syringe pump, an Aurora A inhibitor 6-((4-(2-fluoro-3-(trifluoromethyl)benzoyl)piperazin-1-yl)methyl)-N-1H-pyrazol-3-ylpyrazin-2-amine was administered by intravenous drip infusion for 48 hours. After the administration, the collected cancer graft and the skin specimen were frozen and stored in liquid nitrogen.

Next, the total RNA was isolated from the collected cancer graft and formed into cDNA, which was checked for the expression change of the PD marker candidate genes. In addition to 5 genes identified in culture cells, Aurora A (AurA) and Aurora B (AurB) were also investigated as candidate genes. The primer probe sets for Aurora A and B are as follows. For every primer and probe, a human gene was used as the template.

**[Table 2]**

| Gene | Forward primer | Reverse primer | Probe |
|---|---|---|---|
| AurA | AATCTGGAGGCAAGG TTCGA (SEQ ID NO 16) | GTGAAGACACCATGC CTAGCAC (SEQ ID NO 17) | TGCAGCCGCCCCGTC AGC (SEQ ID NO 18) |
| AurB | GGACCTAAAGTTCCC CGCTT (SEQ ID NO 19) | CCTGAGCAGTTTGGA GATGAGG (SEQ ID NO 20) | TGTGCCCACGGGAGC CCAG (SEQ ID NO 21) |

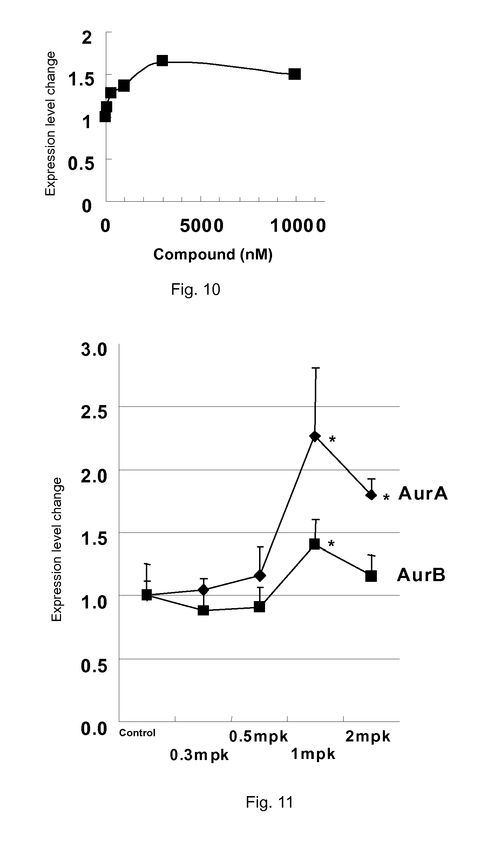

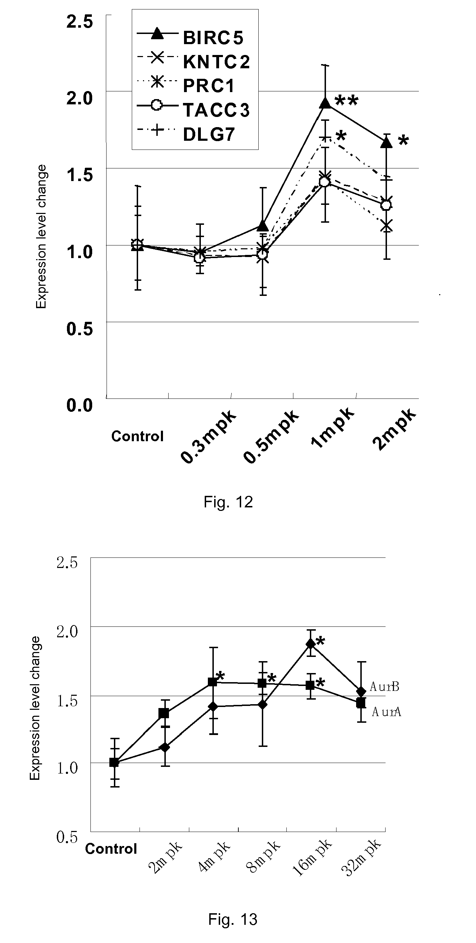

As in Figs. 11 and 12, it has been confirmed that, even in the case where the inhibitor was administered to the rat, the expression level of the PD marker candidate genes increased. It has also been confirmed that the peak of the expression change was at 1 mpk. It has further been confirmed that the expression of BIRC5 and DLG7 significantly increased. Accordingly, when 6-((4-(2-fluoro-3-(trifluoromethyl)benzoyl)piperazin-1-yl)methyl)-N-1H-pyrazol-3-ylpyrazin-2-amine is administered as a pharmaceutical agent, the pharmacological efficacy thereof can be predicted by checking the expression level of the PD marker candidate gene group in the test tissue.

### Example 4

### (Expression Change of PD Marker Candidate Genes in skin in case of continuous administration of Aurora A inhibitor to rat)

The same rat skin as that used in Example 3 was checked for the expression change of PD marker candidate genes through real-time PCR. The primers of the candidate genes in the rat are as shown below. For every primer and probe, a rat gene was used as the template. The probe was modified with FAM/TAMRA.

**[Table 3]**

| Gene | Forward primer | Reverse primer | Probe |
|---|---|---|---|
| AurA | GATCCAGTCCCACCT GCG (SEQ ID NO 22) | CTGGTGGCGTCATGG AAATA (SEQ ID NO 23) | CACCCCAACATCCTCA GGCTGTATGG (SEQ ID NO 24) |
| AurB | TTGCGGACTTTGGCTG GT (SEQ ID NO 25) | GGTGCCGCACATGGT CTT (SEQ ID NO 26) | TGCATGCCCCATCCCT GAGGAG (SEQ ID NO 27) |
| BIRC5 | GGAACCGGATGACAA CCCTA (SEQ ID NO 28) | TTGACTGTAAGGAAG GCGCA (SEQ ID NO 29) | AGAGGAGCATAGGAA GCACTCCCCTGG (SEQ ID NO 30) |
| DLG7 | GGAACAGTGCCATCT ACCACAA (SEQ ID NO 31) | TCCCCTTCTTTCTGAT CCGTT (SEQ ID NO 32) | AAAGCCAGTCACAAG AGCCACCAATGA (SEQ ID NO 33) |
| KNTC2 | TCGGTTTCCAGCTGTG GTG (SEQ ID NO 34) | AGGCCAGGTTTATTG AGGTCC (SEQ ID NO 35) | CCGCCTCTCTATGCAG GAGTTAAGATCCC (SEQ ID NO 36) |
| PRC1 | GCATATCCATCTGTCG GAAGG (SEQ ID NO 37) | TTATCTTCCTCAAATG GCTTGACTT (SEQ ID NO 38) | CTGAGCACCCTGTGC AGCGAGCTA (SEQ ID NO 39) |
| TACC3 | TGCCTTCCTCAGGCTT GC (SEQ ID NO 40) | AGAGATCGCCCAAGT GCG (SEQ ID NO 41) | TGGAGAGCCAGTGCC TCTGCTTGG (SEQ ID NO 42) |

As in Figs. 13 to 16, it has been clarified that, even in the rat skin, the expression level of the candidate genes significantly increased owing to administration of trans-4-(3-chloro-2-fluorophenoxy)-1-((6-(1,3-thiazol-2-ylamino)pyridin-2-yl)methyl)cyclohexanecarboxylic acid. Accordingly, it has been confirmed that, not only in a cancer graft but also in a skin, the effect of an Aurora A inhibitor can be predicted. Fig. 13 shows the expression level change of Aurora A and Aurora B; Fig. 14 shows the expression level change of BIRC5, PRC1 and TACC3; Fig. 15 shows the expression level change of DLG7; and Fig. 16 shows the expression level change of KNTC2.

The above result confirm that the PD marker candidate gene group increased the expression level in any of culture cells, and the cancer tissue and the skin of cancer-transplanted model animal by administration of an Aurora A-specific inhibitor thereto, and they are useful as a PD marker.

### Example 5

### (Increase in Aurora B (Thr232) Phosphorylation specific to Aurora A-deleted HeLa S3 cells by siRNA)

The following test is for confirming the effect in knockdown by siRNA of Aurora A and B in synchronous or asynchronous HeLa S3 cells.

### 1. Analysis of phospho-Aurora B in G1/S stage synchronous cultivation by double-thymidine block:

First, HeLa S3 cells were planted in a 6-well culture tray to be 2.5 × 10⁵ cells/well, and incubated therein overnight at 37°C in 5 % CO₂. Next, at 18:00 on the next day, 200 mM thymidine solution was added to the medium to be a final concentration of 2 mM, followed by further incubation for 16 hours. Further, at 10:00 on the next day, the medium was rinsed three times with PBS, and then exchanged with a fresh medium. In this state, luciferase control (Luc), Aurora A (A1, A2) or Aurora B (B1, B2) siRNA was transfected in each well to be a final concentration of 50 nM, using Oligofectamine (Invitrogen), and then this was further incubated for 8 hours. Next, at 18:00, second thymidine was added to be 2 mM, followed by incubation for 16 hours, and at 10:00 on the next day, the medium was rinsed three times with PBS and exchanged with a fresh medium. As a result, the cells were released from synchronization.

After thus released from synchronization, this was sampled at intervals on 0, 8, 10, 12, 14 and 16 hours and checked for expression of phosphorylated Aurora B according to western blotting with a phospho-Aurora B antibody (Cell Signaling Technology).

### 2. Analysis for knockdown of Aurora A and B in asynchronous culture:

First, HeLa S3 cells were planted in a 6-well culture tray to be 5 × 10⁵ cells/well, and incubated therein overnight at 37°C in 5 % CO₂. Next, on the next day, luciferase control (Luc), Aurora A (A2) or Aurora B (B2) siRNA was transfected in each well to be a final concentration of 50 nM, using Oligofectamine (Invitrogen). Next, after 24 hours, the cells were peeled with trypsin and collected, and a half thereof were analyzed for cell cycle through flow cytometry, and the remaining half thereof were analyzed according to western blotting with mitosis-specific marker candidate (Aurora A, Aurora B, phospho-Aurora B, Survivin, Cyclin B1, Plk1).

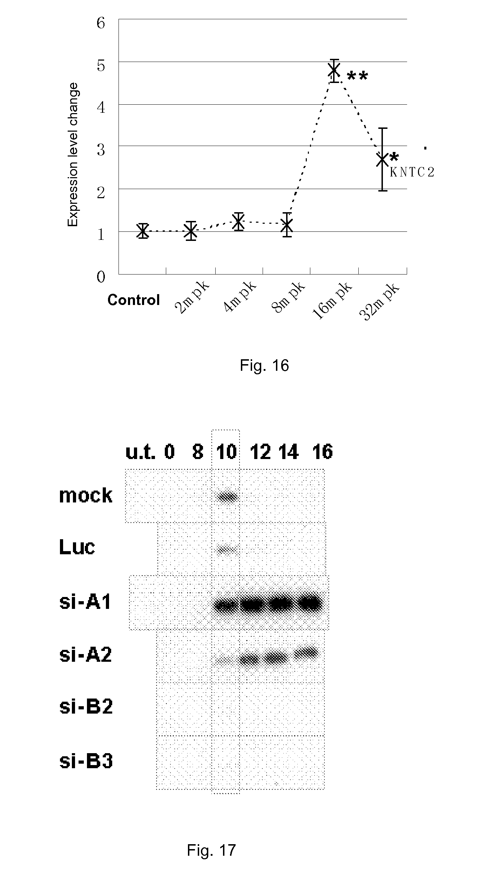

As in Fig. 17, the synchronous HeLa S3 cells generally pass through the M stage in 9 to 10 hours after the synchronization release, and with that, activation (phosphorylation) of Aurora B is detected (mock and Luc). However, in the cells with Aurora A knockdown by siRNA, promotion and maintenance of phosphorylation of Aurora B (Thr232), or that is, mitotic delay was detected (si-A1 and si-A2). On the other hand, in Aurora B knockdown, no phosphorylation itself of Aurora B occurred at all (si-B1 and si-B2).

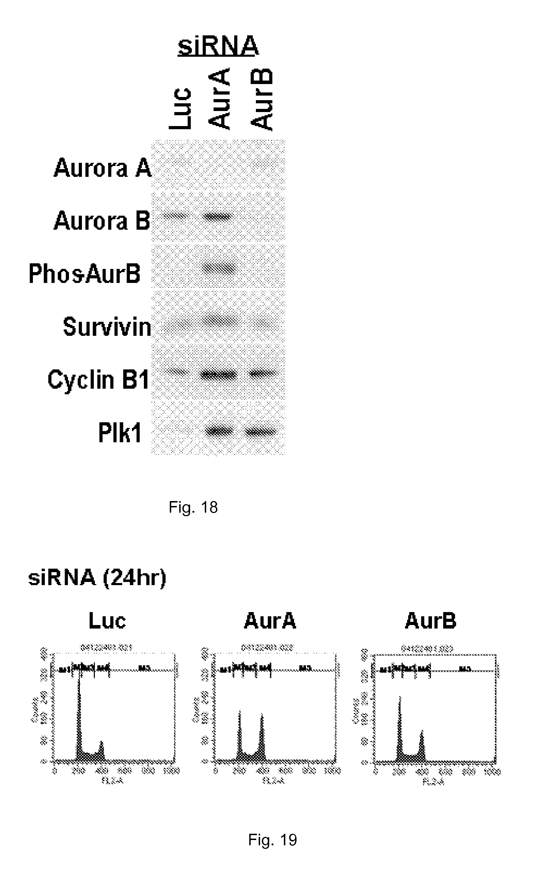

As in Fig. 18, it has been found that, in general, phosphorylation of Aurora B is not detected in the asynchronous HeLa S3 cells, but phospho-Aurora B is detected only in Aurora A siRNA treatment (phos-AurB). As in Fig. 19, it has been found that, in this stage, the proportion of the M-stage cells increased through cell cycle analysis.

Further, some other M-stage marker candidates in the same cell extract were analyzed through western blotting. As in Fig. 18, it has been found these genes were detected in the asynchronous cells and their expression increase magnification in the M-stage was not so large (Survivin and Cyclin B1), or were detected in any knockdown Aurora A or B, and therefore could not be used as a marker specific to Aurora A inhibition (Plk1).

From the above, it has been clarified that Aurora B (Thr232) phosphorylation increases in the cells where Aurora A was specifically deleted with siRNA, and a possibility has been confirmed that phospho-Aurora B (Thr232) may be used as a marker specific to Aurora A inhibition.

### Example 6

### (Search for pharmacodynamic marker of Aurora A-selective inhibitor)

The following test is for confirming the concentration-dependent induction of Aurora B (Thr232) phosphorylation with an Aurora A-selective inhibitor.

First, asynchronously-cultured HeLa S3 cells were planted in an amount of 1 × 10⁶ cells, and incubated overnight at 37°C in 5 % CO₂. At 10:00 on the next day, the medium was exchanged with a medium to which 6-((4-(3-chloro-2-fluorobenzoyl)piperazin-1-yl)methyl)-N-thiazol-2-ylpyridin-2-amine had been added to be a final concentration of 0, 30, 100, 300, 1000, 3000, 10000, 30000 nM, and then, after 8 hours and 24 hours, the cells were peeled with trypsin and collected. A half of the cells was analyzed by western blotting; 1/4 of the cells were analyzed for cell cycle through flow cytometry; and the last remaining ones were analyzed for phosphorylation of histone H3 (Ser10). The cell samples processed in the same manner were analyzed for expression profiling through TaqMan-PCR.

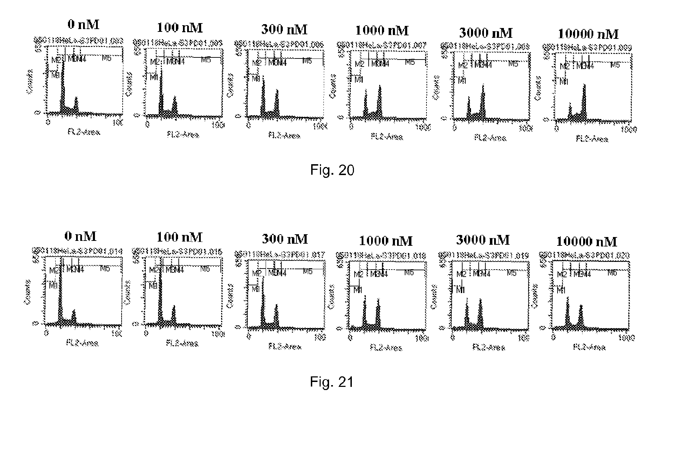

As in Fig. 20 (8 hours) and Fig. 21 (24 hours), the proportion of the M-stage began to increase on and from around 300 nM and the M-stage was kept up to around 10 µM, as the effect of the Aurora A-selective inhibitor.

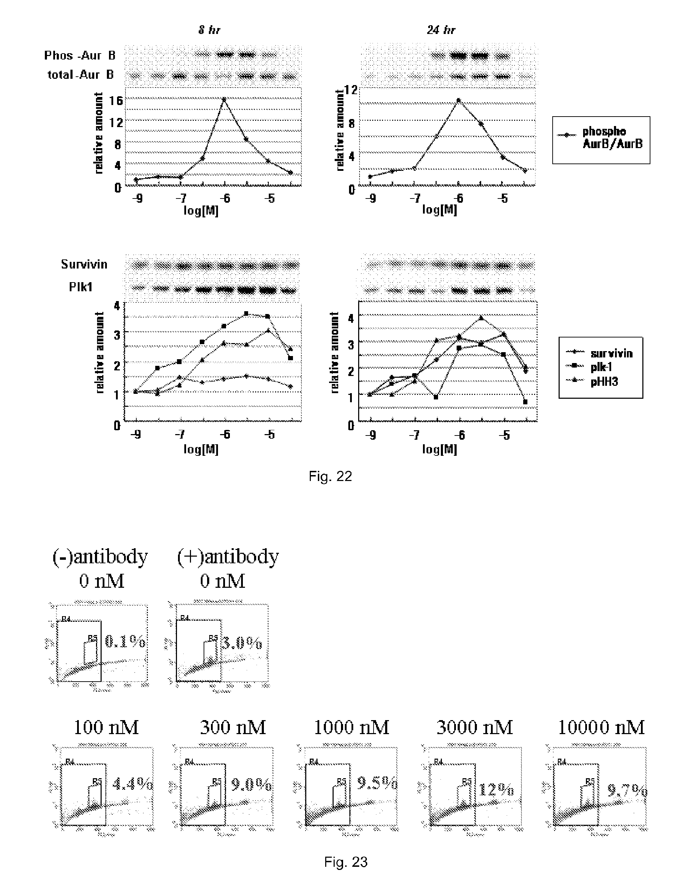

In this case, as in Fig. 22, it has been found that the expression of phospho-Aurora B (Thr232) increased dependently on the administration concentration of the Aurora A-selective compound in any exposure period of time of 8 hours and 24 hours. In this connection, it is considered that the phosphorylation depression at 30 µM or more would be because of Aurora B inhibition. On the other hand, it has been confirmed that the compound administration did not change so much the expression level of Aurora B, and the ratio of phospho-Aurora B/Aurora B increased up to at most 16 times for the exposure period of 8 hours and up to 10 times for the exposure period of 24 hours, based on the compound concentration 0 nM of 1. As opposed to this, the expression of Survivin and Plk1 was admitted in the background with no compound addition, and the expression induction window was at most 3.5 times and was small as compared with that of Phospho-Aurora B. As in Fig. 23, it has been clarified that Histone H3 (Ser10) phosphorylation could not take a large window similarly in flow cytometry.

From the above, it has been clarified that like the case of Aurora A knockdown by siRNA, phospho-Aurora B (Thr232) is specifically induced by administration of the Aurora A-selective compound. Further, the induction increased in correlation with the pharmacological efficacy of the mitotic delay by Aurora A inhibition. However, in the concentration range (> 30 µM) in which Aurora B inhibition begins, the phosphorylation of Aurora B is again inhibited, and it has been confirmed that this can be utilized as a pharmacodynamic marker for Aurora A inhibition.

### Example 7

### (Aurora A inhibition specificity of Aurora B (Thr232) phosphorylation)

The following test is for confirming the Aurora A inhibition specificity of phospho-Aurora B (Thr232), using an Aurora B inhibitor and a pan-Aurora inhibitor (A/B-dual inhibitor).

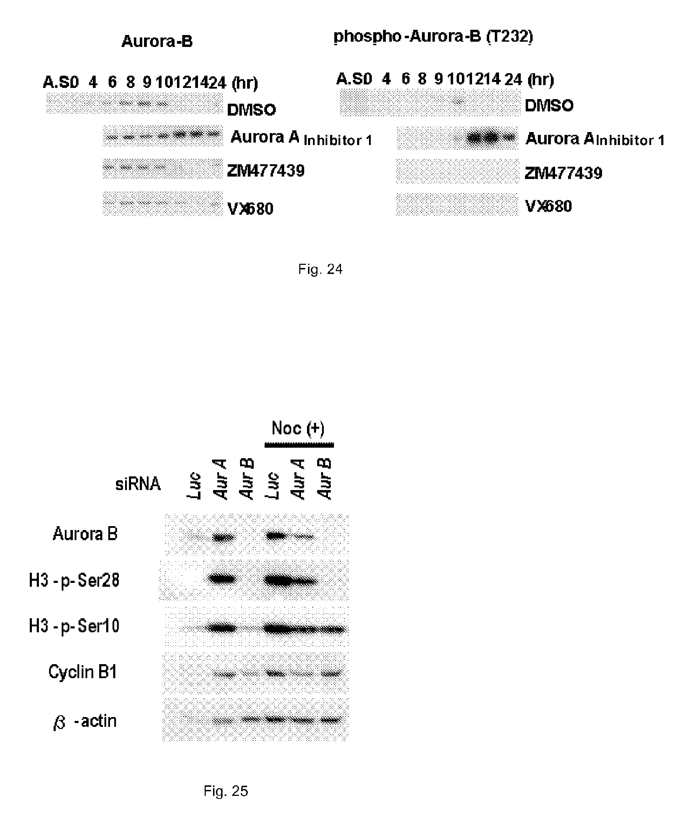

First, HeLa S3 cells were synchronized at the G1/S boundary by the above-mentioned double-thymidine block, and after 4 hours from the start of release, a control (DMSO, final 0.5 %), an Aurora A-selective inhibitor 6-((4-(2,3-difluorobenzoyl)piperazin-1-yl)methyl)-N-thiazol-2-ylpyridin-2-amine (in Fig. 24, this is shown as "Aurora A Inhibitor 1"), an Aurora B inhibitor ZM477439 (J. Cell. Biol., Vol. 161, p. 267, 2003) or a pan-Aurora inhibitor VX-680 was added in an amount of 300 nM, followed by sampling at regular intervals for 0 to 24 hours. Next, the samples were analyzed for total Aurora B and phospho-Aurora B (Thr232) expression by western blotting.

As in Fig. 24, the Aurora B expression gradually increased over the M-stage, but did not change so much. On the other hand, phospho-Aurora B increased temporarily only in 9 to 10 hours over the M-stage. After the cells passed through the M-stage, both Aurora B and phospho-Aurora B greatly reduced. As opposed to this, the cells processed with the Aurora A-selective inhibitor expressed Aurora B to a high level even after 10 hours, and phospho-Aurora B remarkably increased. However, in the cells processed with the Aurora B inhibitor or the pan-Aurora inhibitor, Aurora B was detected on the same level as that in the control, but no phospho-Aurora B was detected at all.

From the above, it has been confirmed that Aurora B (Thr232) phosphorylation is almost completely inhibited by any of pan-Aurora inhibitor, Aurora B inhibitor or Aurora B siRNA treatment, and could not be detected. Accordingly, it has been clarified that Aurora B is a marker specific to Aurora A inhibition.

### Example 8

### (Increase in phosphorylation of histone H3 (Ser28) by Aurora A-selective inhibitor in asynchronous HeLa S3 cells)

The following test is for confirming as to whether or not histone H3 (Ser28) phosphorylation can be used as a PD marker specific to Aurora A inhibition, like phospho-Aurora B.

First, like in Example 5, Luc, Aurora A (A2) or Aurora B (B2) siRNA was transfected in asynchronously-cultured HeLa S3 cells, which were then checked for phosphorylation of histone H3 Ser 28 and Ser 10 by western blotting. The concentration-dependent induction of an Aurora A-selective inhibitor was compared with that of phospho-Aurora B (Thr232), and at the same time, the detectability with a pan-Aurora inhibitor was also checked.

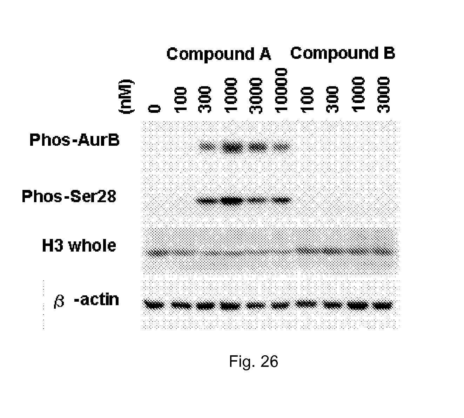

As in Fig. 25, it has been found that the phosphorylation of histone H3 (Ser28) by Aurora A knockdown by siRNA increased like that of Ser10. However, in Aurora B knockdown, Ser10 phosphorylation still remained, but Ser28 phosphorylation almost completely disappeared. From this, it is considered that the Ser28 phosphorylation could be a marker induced specifically to Aurora A inhibition, like that of phospho-Aurora B. Further, as in Fig. 26, the concentration-dependent induction of the Aurora A-selective inhibitor is recognized in nearly the same concentration range as that of phospho-Aurora B, and the pan-Aurora inhibitor did not have a phosphorylation band at all. Accordingly, it is considered that phospho-histone H3 (Ser28) can be used as a PD marker like phospho-Aurora B (Thr232). In Fig. 26, Compound A is 6-((4-(2,3-difluorobenzoyl)piperazin-1-yl)methyl)-N-thiazol-2-ylpyridin-2-amine; and Compound B is VX-680.

### INDUSTRIAL APPLICABILITY

The invention has made it possible to obtain a gene marker and a protein marker capable of detecting whether or not an Aurora A inhibitor may act in a living body in an Aurora A-specific manner when the Aurora A inhibitor is administered to the living body; and based on the marker expression as an index, the pharmacological efficacy of an Aurora A inhibitor can be predicted and determined in a simplified manner and in a noninvasive manner.

Accordingly, in medicinal administration or clinical development of an Aurora A inhibitor, it has become possible to determine the pharmacological efficacy of the inhibitor in a simplified manner and in a noninvasive manner, and the invention is useful for suitable medicinal administration and rapid drug development studies.

### BRIEF DESCRIPTION OF THE DRAWINGS

[Fig. 1] This shows the change in the expression level of BIRC5 gene in inhibition of Aurora A expression by siRNA. In the drawing, the white bar shows the result of treatment with Aurora A-siRNA; and the black bar shows the result of treatment with Aurora B-siRNA.
[Fig. 2] This shows the change in the expression level of PRC1 gene in inhibition of Aurora A expression by siRNA. In the drawing, the white bar shows the result of treatment with Aurora A-siRNA; and the black bar shows the result of treatment with Aurora B-siRNA.
[Fig. 3] This shows the change in the expression level of DLG7 gene in inhibition of Aurora A expression by siRNA. In the drawing, the white bar shows the result of treatment with Aurora A-siRNA; and the black bar shows the result of treatment with Aurora B-siRNA.
[Fig. 4] This shows the change in the expression level of TACC3 gene in inhibition of Aurora A expression by siRNA. In the drawing, the white bar shows the result of treatment with Aurora A-siRNA; and the black bar shows the result of treatment with Aurora B-siRNA.
[Fig. 5] This shows the change in the expression level of KNTC2 gene in inhibition of Aurora A expression by siRNA. In the drawing, the white bar shows the result of treatment with Aurora A-siRNA; and the black bar shows the result of treatment with Aurora B-siRNA.
[Fig. 6] This shows the Aurora A inhibitor concentration and the change in the expression level of BIRC5 gene, in HeLa-S3 cells treated with Aurora A inhibitor.

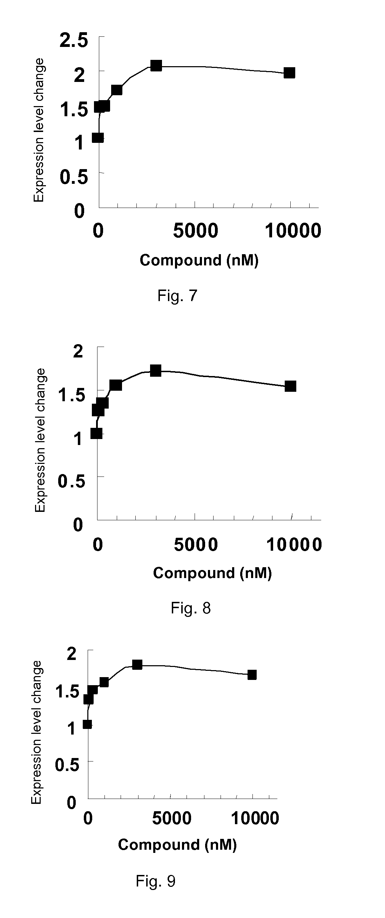

[Fig. 7] This shows the Aurora A inhibitor concentration and the change in the expression level of KNTC2 gene, in HeLa-S3 cells treated with Aurora A inhibitor.
[Fig. 8] This shows the Aurora A inhibitor concentration and the change in the expression level of PRC1 gene, in HeLa-S3 cells treated with Aurora A inhibitor.
[Fig. 9] This shows the Aurora A inhibitor concentration and the change in the expression level of TACC3 gene, in HeLa-S3 cells treated with Aurora A inhibitor.
[Fig. 10] This shows the Aurora A inhibitor concentration and the change in the expression level of DLG7 gene, in HeLa-S3 cells treated with Aurora A inhibitor.
[Fig. 11] This shows the change in the expression level of Aurora A and Aurora B genes in rats administered with Aurora A inhibitor. In the drawing, * means P < 0.05 as compared with the control.
[Fig. 12] This shows the change in the expression level of BIRC5, KNTC2, PRC1, TACC3 and DLG7 genes in rats administered with Aurora A inhibitor. In the drawing, * means P < 0.05 as compared with the control; and ** means P < 0.01.
[Fig. 13] This shows the change in the expression level of Aurora A and Aurora B genes in rats administered with Aurora A inhibitor. In the drawing, * means P < 0.05 as compared with the control.

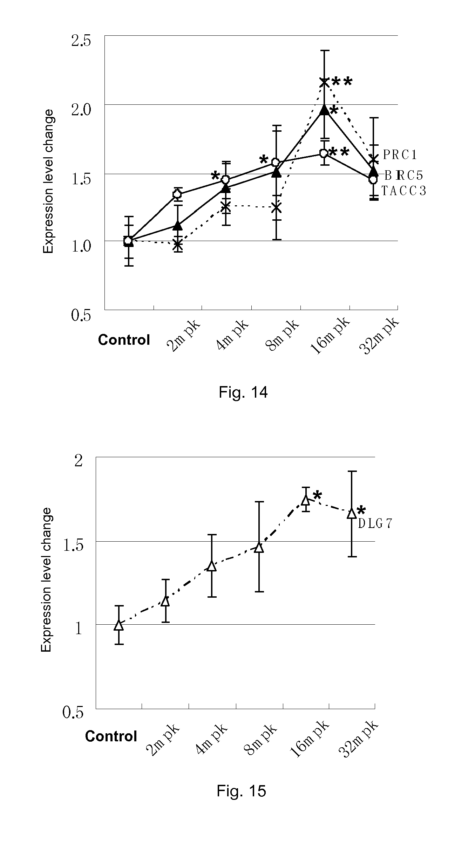

[Fig. 14] This shows the change in the expression level of BIRC5, PRC1 and TACC3 genes in rats administered with Aurora A inhibitor. In the drawing, * means P < 0.05 as compared with the control; and ** means P < 0.01.
[Fig. 15] This shows the change in the expression level of DLG7 gene in rats administered with Aurora A inhibitor. In the drawing, * means P < 0.05 as compared with the control.
[Fig. 16] This shows the change in the expression level of KNTC2 gene in rats administered with Aurora A inhibitor. In the drawing, * means P < 0.05 as compared with the control; and ** means P < 0.01.
[Fig. 17] This shows the result of investigation of the phosphorylation condition of Aurora B (Thr232) in synchronous HeLa-S3 cells where the expression of Aurora A or Aurora was inhibited by siRNA.
[Fig. 18] This shows the result of investigation of the phosphorylation condition of gene marker candidate genes in asynchronous HeLa-S3 cells where the expression of Aurora A or Aurora was inhibited by siRNA.
[Fig. 19] This shows the result of cell cycle analysis in asynchronous HeLa-S3 cells where the expression of Aurora A or Aurora was inhibited by siRNA.
[Fig. 20] This shows the relationship between the administration concentration and the cell cycle in 8 hours after administration of Aurora A inhibitor to HeLa-S3 cells.
[Fig. 21] This shows the relationship between the administration concentration and the cell cycle in 24 hours after administration of Aurora A inhibitor to HeLa-S3 cells.
[Fig. 22] This shows the relationship between the administration concentration of Aurora A inhibitor to HeLa-S3 cells and the expression level (protein) of phospho-Aurora B (Thr232), Plk1 and Survivin in the cells.
[Fig. 23] This shows the relationship between the administration concentration of Aurora A inhibitor to HeLa-S3 cells and phosphorylation of histone H3 (Ser10) in the cells.
[Fig. 24] This shows the result of the expression level change with time of Aurora B and phospho-Aurora B in administration of Aurora A inhibitor, Aurora B inhibitor or pan-Aurora inhibitor.
[Fig. 25] This shows the result of phosphorylation of histone H3 (Ser28) or histone H3 (Ser10) in cells where of Aurora A or Aurora B expression was inhibited by siRNA.
[Fig. 26] This shows the relationship between the administration concentration of Aurora A inhibitor or pan-Aurora inhibitor and the phosphorylation of histone H3 (Ser28) or Aurora B (Thr232).

## Claims

1. A gene marker for predicting or determining the pharmacological efficacy of an Aurora A inhibitor, wherein the gene is at least one gene selected from a group consisting of Aurora B, Histone H3, BIRC5, PRC1, DLG7, TACC3 and KNTC2.

2. The gene marker as claimed in claim 1, wherein the Aurora A inhibitor is an anticancer agent.

3. A gene marker for determining the healing result of cancer, wherein the gene is at least one gene selected from a group consisting of Aurora B, Histone H3, BIRC5, PRC1, DLG7, TACC3 and KNTC2.

4. The gene marker as claimed in claim 3, wherein the cancer healing is by an Aurora A inhibitor.

5. A cancer diagnostic kit containing a PCR primer capable of detecting at least one gene selected from a group consisting of Aurora B, Histone H3, BIRC5, PRC1, DLG7, TACC3 and KNTC2.

6. A DNA microarray for use for predicting or determining the effect of an Aurora A inhibitor, which comprises at least one gene selected from a group consisting of Aurora B, Histone H3, BIRC5, PRC1, DLG7, TACC3 and KNTC2 or a partial nucleotide of the gene.

7. A protein marker for predicting or determining the pharmacological efficacy of an Aurora A inhibitor, wherein the protein is at least one protein selected from a group consisting of Aurora B, Histone H3, BIRC5, PRC1, DLG7, TACC3 and KNTC2.

8. The protein marker as claimed in claim 7, wherein the Aurora A inhibitor is an anticancer agent.

9. A protein marker for determining the healing result of cancer, wherein the protein is at least one protein selected from a group consisting of Aurora B, Histone H3, BIRC5, PRC1, DLG7, TACC3 and KNTC2.

10. The protein marker as claimed in claim 9, wherein the cancer healing is by an Aurora A inhibitor.

11. A cancer diagnostic kit containing an antibody capable of detecting the protein marker of any one of claims 7 to 10.

12. An antibody array for use for predicting or determining the effect of an Aurora A inhibitor, which comprises an antibody to at least one protein selected from a group consisting of Aurora B, Histone H3, BIRC5, PRC1, DLG7, TACC3 and KNTC2.

13. A method for predicting or determining the effect of an Aurora A inhibitor comprising:
a detection step of detecting at least one gene selected from a group consisting of Aurora B, Histone H3, BIRC5, PRC1, DLG7, TACC3 and KNTC2 in a tissue derived from a test subject to which an Aurora A inhibitor has been administered, and
a comparison step of comparing the expression level before and after the Aurora A inhibitor administration.

14. The method for predicting or determining the effect of an Aurora A inhibitor as claimed in claim 13, wherein the detection means for the detection is a PCR or DNA microarray.

15. A method for predicting or determining the effect of an Aurora A inhibitor comprising:
a detection step of detecting at least one protein selected from a group consisting of Aurora B, Histone H3, BIRC5, PRC1, DLG7, TACC3 and KNTC2 in a tissue derived from a test subject to which an Aurora A inhibitor has been administered, and
a comparison step of comparing the expression level before and after the Aurora A inhibitor administration.

16. The method for predicting or determining the effect of an Aurora A inhibitor as claimed in claim 15, wherein the detection means for the detection is an anti-Aurora B antibody, an anti-Histone H3 antibody, an anti-BIRC5 antibody, an anti-PRC1 antibody, an anti-DLG7 antibody, an anti-TACC3 antibody or an anti-KNTC2 antibody.

17. The method for predicting or determining the effect of an Aurora A inhibitor as claimed in any one of claims 13 to 16, wherein the Aurora A inhibitor is an anticancer agent.

18. The method for predicting or determining the effect of an Aurora A inhibitor as claimed in any one of claims 13 to 17, wherein the tissue is blood, skin, hair root, oral mucosa, digestive tract, bone marrow or cancer tissue.


SEQUENCE LISTING

<110>  Banyu Pharmaceutical Co.,Ltd.

<120>  Marker gene and protien which estimate the efficacy of a drug or the
diagnosis of Aurora A inhibitor

<130>  BY0168

<150>  JP2006-273945
<151>  2006-10-05

<160>  42

<170>  PatentIn version 3.1

<210>  1
<211>  20
<212>  DNA
<213>  Artificial Sequence

<220>
<223>  Synthetic polynucleotide

<400>  1
ctttctttgg aggcagcagc                                                   20


<210>  2
<211>  20
<212>  DNA
<213>  Artificial Sequence

<220>
<223>  Synthetic polynucleotide

<400>  2
gggcgaatca aatccatcat                                                   20


<210>  3
<211>  23
<212>  DNA
<213>  Artificial Sequence

<220>
<223>  Synthetic polynucleotide

<400>  3
cgcagggctg aagtctggcg taa                                               23


<210>  4
<211>  20
<212>  DNA
<213>  Artificial Sequence

<220>
<223>  Synthetic polynucleotide

<400>  4
tctcatctcc cccattcctg                                                   20


<210>  5
<211>  18
<212>  DNA
<213>  Artificial Sequence

<220>
<223>  Synthetic polynucleotide

<400>  5
actcccaatg tggctggc                                                    18


<210>  6
<211>  24
<212>  DNA
<213>  Artificial Sequence

<220>
<223>  Synthetic polynucleotide

<400>  6
tgcctgagtt cttctacccc cgca                                             24


<210>  7
<211>  27
<212>  DNA
<213>  Artificial Sequence

<220>
<223>  Synthetic polynucleotide

<400>  7
agaattccaa aggttatgac tttgaaa                                          27


<210>  8
<211>  21
<212>  DNA
<213>  Artificial Sequence

<220>
<223>  Synthetic polynucleotide

<400>  8
gccctgtatt tgacaaggca g                                                21


<210>  9
<211>  25
<212>  DNA
<213>  Artificial Sequence

<220>
<223>  Synthetic polynucleotide

<400>  9
aagtttaatc ccgaggctgg tgcca                                            25


<210>  10
<211>  23
<212>  DNA
<213>  Artificial Sequence

<220>
<223>  Synthetic polynucleotide

<400>  10
aagaagtgcg tggaggatta cct                                              23

<210>  11
<211>  20
<212>  DNA
<213>  Artificial Sequence

<220>
<223>  Synthetic polynucleotide

<400>  11
gccttcaggg cttggtacct                                                    20


<210>  12
<211>  22
<212>  DNA
<213>  Artificial Sequence

<220>
<223>  Synthetic polynucleotide

<400>  12
caaggatcac ccaggagggc ca                                                 22


<210>  13
<211>  23
<212>  DNA
<213>  Artificial Sequence

<220>
<223>  Synthetic polynucleotide

<400>  13
gctggagagg agacatcaag aac                                                23


<210>  14
<211>  27
<212>  DNA
<213>  Artificial Sequence

<220>
<223>  Synthetic polynucleotide

<400>  14
cctggttgta gaggtgaaaa agtaatc                                            27


<210>  15
<211>  29
<212>  DNA
<213>  Artificial Sequence

<220>
<223>  Synthetic polynucleotide

<400>  15
tgccagacac atttcttttg gtggtaacc                                          29


<210>  16
<211>  20
<212>  DNA
<213>  Artificial Sequence

<220>
<223>  Synthetic polynucleotide

<400>  16
aatctggagg caaggttcga                                                   20


<210>  17
<211>  22
<212>  DNA
<213>  Artificial Sequence

<220>
<223>  Synthetic polynucleotide

<400>  17
gtgaagacac catgcctagc ac                                                22


<210>  18
<211>  18
<212>  DNA
<213>  Artificial Sequence

<220>
<223>  Synthetic polynucleotide

<400>  18
tgcagccgcc ccgtcagc                                                     18


<210>  19
<211>  20
<212>  DNA
<213>  Artificial Sequence

<220>
<223>  Synthetic polynucleotide

<400>  19
ggacctaaag ttccccgctt                                                   20


<210>  20
<211>  22
<212>  DNA
<213>  Artificial Sequence

<220>
<223>  Synthetic polynucleotide

<400>  20
cctgagcagt ttggagatga gg                                                22


<210>  21
<211>  19
<212>  DNA
<213>  Artificial Sequence

<220>
<223>  Synthetic polynucleotide

<400>  21
tgtgcccacg ggagcccag                                                    19


<210>  22
<211>  18
<212>  DNA
<213>  Artificial Sequence

<220>
<223>  Synthetic polynucleotide

<400>  22
gatccagtcc cacctgcg                                                    18


<210>  23
<211>  20
<212>  DNA
<213>  Artificial Sequence

<220>
<223>  Synthetic polynucleotide

<400>  23
ctggtggcgt catggaaata                                                  20


<210>  24
<211>  26
<212>  DNA
<213>  Artificial Sequence

<220>
<223>  Synthetic polynucleotide

<400>  24
caccccaaca tcctcaggct gtatgg                                           26


<210>  25
<211>  18
<212>  DNA
<213>  Artificial Sequence

<220>
<223>  Synthetic polynucleotide

<400>  25
ttgcggactt tggctggt                                                    18


<210>  26
<211>  18
<212>  DNA
<213>  Artificial Sequence

<220>
<223>  Synthetic polynucleotide

<400>  26
ggtgccgcac atggtctt                                                    18


<210>  27
<211>  22
<212>  DNA
<213>  Artificial Sequence

<220>
<223>  Synthetic polynucleotide

<400>  27
tgcatgcccc atccctgagg ag                                               22

<210>  28
<211>  20
<212>  DNA
<213>  Artificial Sequence

<220>
<223>  Synthetic polynucleotide

<400>  28
ggaaccggat gacaacccta                                                    20


<210>  29
<211>  20
<212>  DNA
<213>  Artificial Sequence

<220>
<223>  Synthetic polynucleotide

<400>  29
ttgactgtaa ggaaggcgca                                                    20


<210>  30
<211>  27
<212>  DNA
<213>  Artificial Sequence

<220>
<223>  Synthetic polynucleotide

<400>  30
agaggagcat aggaagcact cccctgg                                            27


<210>  31
<211>  22
<212>  DNA
<213>  Artificial Sequence

<220>
<223>  Synthetic polynucleotide

<400>  31
ggaacagtgc catctaccac aa                                                 22


<210>  32
<211>  21
<212>  DNA
<213>  Artificial Sequence

<220>
<223>  Synthetic polynucleotide

<400>  32
tccccttctt tctgatccgt t                                                  21


<210>  33
<211>  27
<212>  DNA
<213>  Artificial Sequence

<220>
<223>  Synthetic polynucleotide

<400>  33
aaagccagtc acaagagcca ccaatga                                          27


<210>  34
<211>  19
<212>  DNA
<213>  Artificial Sequence

<220>
<223>  Synthetic polynucleotide

<400>  34
tcggtttcca gctgtggtg                                                   19


<210>  35
<211>  21
<212>  DNA
<213>  Artificial Sequence

<220>
<223>  Synthetic polynucleotide

<400>  35
aggccaggtt tattgaggtc c                                                21


<210>  36
<211>  29
<212>  DNA
<213>  Artificial Sequence

<220>
<223>  Synthetic polynucleotide

<400>  36
ccgcctctct atgcaggagt taagatccc                                        29


<210>  37
<211>  21
<212>  DNA
<213>  Artificial Sequence

<220>
<223>  Synthetic polynucleotide

<400>  37
gcatatccat ctgtcggaag g                                                21


<210>  38
<211>  25
<212>  DNA
<213>  Artificial Sequence

<220>
<223>  Synthetic polynucleotide

<400>  38
ttatcttcct caaatggctt gactt                                            25


<210>  39
<211>  24
<212>  DNA
<213>  Artificial Sequence

<220>
<223>  Synthetic polynucleotide

<400>  39
ctgagcaccc tgtgcagcga gcta                                              24


<210>  40
<211>  18
<212>  DNA
<213>  Artificial Sequence

<220>
<223>  Synthetic polynucleotide

<400>  40
tgccttcctc aggcttgc                                                     18


<210>  41
<211>  18
<212>  DNA
<213>  Artificial Sequence

<220>
<223>  Synthetic polynucleotide

<400>  41
agagatcgcc caagtgcg                                                     18


<210>  42
<211>  24
<212>  DNA
<213>  Artificial Sequence

<220>
<223>  Synthetic polynucleotide

<400>  42
tggagagcca gtgcctctgc ttgg                                              24

SEQUENCE LISTING

<110> Banyu Pharmaceutical Co.,Ltd.

<120> Marker gene and protien which estimate the efficacy of a drug or the
diagnosis of Aurora A inhibitor

<130> BY0168

<150> JP2006-273945
<151> 2006-10-05

<160> 42

<170> PatentIn version 3.1

<210> 1
<211> 20
<212> DNA
<213> Artificial

<220>
<223> Synthetic polynucleotide

<400> 1
ctttctttgg aggcagcagc 20

<210> 2
<211> 20
<212> DNA
<213> Artificial

<220>
<223> Synthetic polynucleotide

<400> 2
gggcgaatca aatccatcat 20

<210> 3
<211> 23
<212> DNA
<213> Artificial

<220>
<223> Synthetic polynucleotide

<400> 3
cgcagggctg aagtctggcg taa 23

<210> 4
<211> 20
<212> DNA
<213> Artificial

<220>
<223> Synthetic polynucleotide

<400> 4
tctcatctcc cccattcctg 20

<210> 5
<211> 18
<212> DNA
<213> Artificial

<220>
<223>  Synthetic polynucleotide

<400>  5
actcccaatg tggctggc                                                    18


<210>  6
<211>  24
<212>  DNA
<213>  Artificial

<220>
<223>  Synthetic polynucleotide

<400>  6
tgcctgagtt cttctacccc cgca                                             24


<210>  7
<211>  27
<212>  DNA
<213>  Artificial

<220>
<223>  Synthetic polynucleotide

<400>  7
agaattccaa aggttatgac tttgaaa                                          27


<210>  8
<211>  21
<212>  DNA
<213>  Artificial

<220>
<223>  Synthetic polynucleotide

<400>  8
gccctgtatt tgacaaggca g                                                21


<210>  9
<211>  25
<212>  DNA
<213>  Artificial

<220>
<223>  Synthetic polynucleotide

<400>  9
aagtttaatc ccgaggctgg tgcca                                            25


<210>  10
<211>  23
<212>  DNA
<213>  Artificial

<220>
<223>  Synthetic polynucleotide

<400>  10
aagaagtgcg tggaggatta cct                                              23


<210>  11
<211>  20

<212>  DNA
<213>  Artificial

<220>
<223>  Synthetic polynucleotide

<400>  11
gccttcaggg cttggtacct                                                    20


<210>  12
<211>  22
<212>  DNA
<213>  Artificial

<220>
<223>  Synthetic polynucleotide

<400>  12
caaggatcac ccaggagggc ca                                                 22


<210>  13
<211>  23
<212>  DNA
<213>  Artificial

<220>
<223>  Synthetic polynucleotide

<400>  13
gctggagagg agacatcaag aac                                                23


<210>  14
<211>  27
<212>  DNA
<213>  Artificial

<220>
<223>  Synthetic polynucleotide

<400>  14
cctggttgta gaggtgaaaa agtaatc                                            27


<210>  15
<211>  29
<212>  DNA
<213>  Artificial

<220>
<223>  Synthetic polynucleotide

<400>  15
tgccagacac atttcttttg gtggtaacc                                          29


<210>  16
<211>  20
<212>  DNA
<213>  Artificial

<220>
<223>  Synthetic polynucleotide

<400>  16
aatctggagg caaggttcga                                                    20

<210> 17
<211> 22
<212> DNA
<213> Artificial

<220>
<223> Synthetic polynucleotide

<400> 17
gtgaagacac catgcctagc ac 22


<210> 18
<211> 18
<212> DNA
<213> Artificial

<220>
<223> Synthetic polynucleotide

<400> 18
tgcagccgcc ccgtcagc 18


<210> 19
<211> 20
<212> DNA
<213> Artificial

<220>
<223> Synthetic polynucleotide

<400> 19
ggacctaaag ttccccgctt 20


<210> 20
<211> 22
<212> DNA
<213> Artificial

<220>
<223> Synthetic polynucleotide

<400> 20
cctgagcagt ttggagatga gg 22


<210> 21
<211> 19
<212> DNA
<213> Artificial

<220>
<223> Synthetic polynucleotide

<400> 21
tgtgcccacg ggagcccag 19


<210> 22
<211> 18
<212> DNA
<213> Artificial

<220>
<223> Synthetic polynucleotide

<400> 22
gatccagtcc cacctgcg 18

<210> 23
<211> 20
<212> DNA
<213> Artificial

<220>
<223> Synthetic polynucleotide

<400> 23
ctggtggcgt catggaaata 20

<210> 24
<211> 26
<212> DNA
<213> Artificial

<220>
<223> Synthetic polynucleotide

<400> 24
caccccaaca tcctcaggct gtatgg 26

<210> 25
<211> 18
<212> DNA
<213> Artificial

<220>
<223> Synthetic polynucleotide

<400> 25
ttgcggactt tggctggt 18

<210> 26
<211> 18
<212> DNA
<213> Artificial

<220>
<223> Synthetic polynucleotide

<400> 26
ggtgccgcac atggtctt 18

<210> 27
<211> 22
<212> DNA
<213> Artificial

<220>
<223> Synthetic polynucleotide

<400> 27
tgcatgcccc atccctgagg ag 22

<210> 28
<211> 20
<212> DNA
<213> Artificial

<220>
<223>  Synthetic polynucleotide

<400>  28
ggaaccggat gacaacccta                                                    20


<210>  29
<211>  20
<212>  DNA
<213>  Artificial

<220>
<223>  Synthetic polynucleotide

<400>  29
ttgactgtaa ggaaggcgca                                                    20


<210>  30
<211>  27
<212>  DNA
<213>  Artificial

<220>
<223>  Synthetic polynucleotide

<400>  30
agaggagcat aggaagcact cccctgg                                            27


<210>  31
<211>  22
<212>  DNA
<213>  Artificial

<220>
<223>  Synthetic polynucleotide

<400>  31
ggaacagtgc catctaccac aa                                                 22


<210>  32
<211>  21
<212>  DNA
<213>  Artificial

<220>
<223>  Synthetic polynucleotide

<400>  32
tccccttctt tctgatccgt t                                                  21


<210>  33
<211>  27
<212>  DNA
<213>  Artificial

<220>
<223>  Synthetic polynucleotide

<400>  33
aaagccagtc acaagagcca ccaatga                                            27


<210>  34
<211>  19

<212>  DNA
<213>  Artificial

<220>
<223>  Synthetic polynucleotide

<400>  34
tcggtttcca gctgtggtg                                                    19

<210>  35
<211>  21
<212>  DNA
<213>  Artificial

<220>
<223>  Synthetic polynucleotide

<400>  35
aggccaggtt tattgaggtc c                                                 21

<210>  36
<211>  29
<212>  DNA
<213>  Artificial

<220>
<223>  Synthetic polynucleotide

<400>  36
ccgcctctct atgcaggagt taagatccc                                         29

<210>  37
<211>  21
<212>  DNA
<213>  Artificial

<220>
<223>  Synthetic polynucleotide

<400>  37
gcatatccat ctgtcggaag g                                                 21

<210>  38
<211>  25
<212>  DNA
<213>  Artificial

<220>
<223>  Synthetic polynucleotide

<400>  38
ttatcttcct caaatggctt gactt                                             25

<210>  39
<211>  24
<212>  DNA
<213>  Artificial

<220>
<223>  Synthetic polynucleotide

<400>  39
ctgagcaccc tgtgcagcga gcta                                              24

<210> 40
<211> 18
<212> DNA
<213> Artificial

<220>
<223> Synthetic polynucleotide

<400> 40
tgccttcctc aggcttgc 18


<210> 41
<211> 18
<212> DNA
<213> Artificial

<220>
<223> Synthetic polynucleotide

<400> 41
agagatcgcc caagtgcg 18


<210> 42
<211> 24
<212> DNA
<213> Artificial

<220>
<223> Synthetic polynucleotide

<400> 42
tggagagcca gtgcctctgc ttgg 24